# EUROPEAN PATENT APPLICATION

(11) **EP 1 776 980 A1**
(43) Date of publication of application: **25.04.2007**
(21) Application number: 06255363.1
(22) Date of filing: 18.10.2006
(51) Int. Cl.: A61M 25/06, A61M 5/158

(54) **Safety infusion set**

(30) Priority: 19.10.2005 US 728124 P
(71) Applicant: Animas Corporation, West Chester PA 19380 (US)
(72) Inventor: Wojcik, Steven E., Shoreline, WA 98177 (US)
(74) Representative: Tunstall, Christopher Stephen

(57) **Abstract**

An infusion set is disclosed. The infusion set includes a cannula hub assembly having a cannula housing body (104) with a distal end and a bottom surface. The set includes an insertion needle assembly (200) having an elongated insertion needle hub adapted to be coupled to the cannula housing body and a cannula having a distal cannula end. The cannula is at least partially slidably disposed within the insertion needle hub. The insertion needle assembly also includes an insertion needle having a distal insertion needle end, wherein the needle extends coaxially through the cannula. The distal cannula end and the distal insertion needle end are adapted to be inserted through the distal end of the cannula housing body. The insertion needle is retractable from the cannula housing body to be at least substantially disposed within the insertion needle hub. A method of inserting the cannula through a patient's skin is also disclosed.

## Description

### CROSS REFERENCE TO RELATED APPLICATION

The present application claims priority from U.S. Provisional Patent Application Serial No. 60/728,124, filed on October 19, 2005.

### FIELD OF THE INVENTION

The present invention relates to an infusion set that reduces the risks of accidental needle sticks before and after percutaneous insertion of a cannula into a patient.

### BACKGROUND OF THE INVENTION

Frequent or continuous subcutaneous injection of medication is often accomplished through the use of an infusion set or injection port which may remain in place for several days. In the case of frequent injections, this reduces the need to constantly puncture the skin and thereby reduce the chance of infection and the formation of scar tissue. For continuous subcutaneous delivery of medication, such as commonly used with portable insulin pumps, an infusion set is often used to provide a method of temporarily detaching the pump and fluid line for activities such as dressing or bathing. It is also desirable in this instance to detach the fluid line from the pump as close to the injection site as possible, leaving a relatively small component attached to the patient's skin to minimize the interference during dressing, bathing or other activities.

Most infusion sets consist of a soft flexible cannula extending from a housing which can be attached to the skin. The end of the cannula within the housing is sealed by an elastomeric septum. Initially, a sharp insertion needle attached to an insertion needle hub or handle passes coaxially through the septum and cannula. The sharp end of the needle extends past the distal end of the cannula, allowing the insertion needle and cannula to easily penetrate skin. Once the insertion needle and cannula are inserted subcutaneously, the cannula housing body is attached to the surface of the skin with medical tape or an integral adhesive pad. The insertion needle is then withdrawn from the cannula and septum and replaced with a tubing hub which attaches to the housing. The tubing hub includes a length of flexible tubing to connect to the reservoir of an insulin infusion pump at one end and a short hollow needle at the other end which penetrates the septum. Once connected, insulin can flow from the infusion pump, through the cannula, and into the subcutaneous tissue.

While devices for this purpose are well known in the art, they all have limitations making them less than ideal in practice, particularly with the method used to insert the cannula subcutaneously. Conventional angled infusion sets have the disadvantage of a relatively long needle necessary to position the cannula tip at a shallow angle deep enough below the skin's surface to reliably deliver the medication into subcutaneous tissue, yet not be easily dislodged. The long needle and cannula must be carefully inserted to avoid bending the needle or allow the needle to contact underlying muscle tissue. This is made more difficult if the infusion site cannot be viewed directly. Also, while the angle of insertion is not critical, care must be taken to avoid inserting the insertion needle and cannula too deep into the muscle tissue, causing pain, or at too shallow of an angle where the insulin may not be absorbed. The long needle may also be intimidating to some users, especially children.

The infusion sets are typically supplied sterile and care must be taken to avoid contaminating the insertion needle and cannula prior to insertion. The user must remove the infusion set from the sterile package, then position the set at the correct angle without allowing the insertion needle and cannula to contact the skin except at the prepared infusion site. This procedure requires the user to hold the main body of the infusion set above the skin, often in an area of the body that cannot be viewed directly, insert the insertion needle and cannula fully, remove adhesive liner papers, press the base of the set against the skin, then smooth the adhesive pad to skin without wrinkles. This process requires a fair amount of dexterity and can be especially difficult for children.

Another problem with all types of existing infusion sets is that the used insertion needle must be removed and discarded. While some infusion sets have provisions to bend the needle after removal from the cannula to reduce the risk of an accidental needle stick, it would be beneficial to provide an infusion set in which the insertion needle and cannula would not be exposed either before or after the insertion of the infusion set to reduce the risk of contamination or accidental needle sticks.

### SUMMARY OF THE INVENTION

Briefly, the present invention provides an infusion set comprising a cannula hub assembly including a cannula housing body having a distal end, a bottom surface and an insertion needle assembly. The insertion needle assembly includes an elongated insertion needle hub adapted to be coupled to the cannula housing body and a cannula having a distal cannula end. The cannula is at least partially slidably disposed within the insertion needle hub. The insertion needle assembly also includes an insertion needle having a distal insertion needle end. The needle extends coaxially through the cannula. The distal cannula end and the distal insertion needle end are adapted to be inserted through the distal end of the cannula housing body, and the insertion needle is retractable from the cannula housing body to be at least substantially disposed within the insertion needle hub.

Additionally, the present invention also includes a method of percutaneously inserting a cannula of an infusion set through a patient's skin. The infusion set comprises an insertion needle hub releasably coupled to a cannula hub assembly. The cannula has an insertion needle disposed therethrough and the needle extends slidably within the insertion needle assembly and the cannula hub assembly. The method comprises the steps of attaching at least a portion of a base portion of the infusion set to the patient's skin at an insertion site; advancing the cannula and the insertion needle through the cannula hub assembly and through the patient's skin; and retracting the insertion needle into the insertion needle assembly while leaving the cannula in the patient's skin.

Further, the present invention provides a safety needle assembly for an infusion set. The safety needle assembly comprises a cannula housing body having a bore extending therethrough and an insertion needle hub body releasably connected to the cannula housing body. A cannula havs a distal cannula end. The cannula is at least partially slidably disposed within the insertion needle hub. An insertion needle has a distal insertion needle end. The needle extends coaxially through the cannula. The assembly further includes means for advancing the insertion needle and the cannula distally through the cannula housing body and for proximally retracting the insertion needle such that the insertion needle is entirely disposed within the insertion needle hub assembly.

### BRIEF DESCRIPTION OF THE DRAWINGS

The foregoing summary, as well as the following detailed description a preferred embodiment of the invention, will be better understood when read in conjunction with the appended drawings, which are incorporated herein and constitute part of this specification. For the purposes of illustrating the invention, there are shown in the drawings an exemplary embodiment of the present invention. It should be understood, however, that the invention is not limited to the precise arrangements and instrumentalities shown. In the drawings, the same reference numerals are employed for designating the same elements throughout the several figures. In the drawings:
Fig. 1 is a perspective view of an infusion set according to a first exemplary embodiment of the present invention, with a plunger retracted and an insertion needle assembly coupled to a cannula housing assembly;
Fig. 2 is an enlarged perspective view of the cannula housing assembly of Fig. 1;
Fig. 3 is a top plan view of an exemplary housing body of the infusion set of Fig. 1;
Fig. 4 is a side elevational view, in section, of the cannula housing assembly taken along lines 4--4 of Fig. 3;
Fig. 5 is a proximal end elevational view of the cannula housing assembly, taken along lines 5--5 of Fig. 3;
Fig. 6 is an exploded perspective view of a cannula assembly of the exemplary infusion set of Fig. 1;
Fig. 7 is a side elevational view, in section, of the cannula assembly of Fig. 6;
Fig. 8 is an exploded perspective view of an insertion hub assembly of the exemplary infusion set of Fig. 1;
Fig. 9 is an elevational view, in section, taken along lines 9--9 of Fig. 8;
Fig. 10 is a side elevational view, in section, of the exemplary infusion set of Fig. 1 having been attached to a patient's skin;
Fig. 11 is an elevational view, in section, taken along lines 11--11 of Fig. 10;
Fig. 12 is a perspective view of the cannula housing assembly with the tubing hub assembly being coupled to the cannula housing assembly;
Fig. 13 is an elevational view, in section, taken along lines 13--13 of Fig. 12;
Fig. 14 is a perspective view of an insertion needle being advanced into the patient;
Fig. 15 is a side elevational view, in section, of the infusion set with the insertion needle having been advanced into the patient's skin;
Fig. 16 is a side elevational view, in section, of the infusion set with the insertion needle having been withdrawn from the patient's skin;
Fig. 17 is a perspective view of infusion set with the insertion needle having been withdrawn from the patient's skin;
Fig. 18 is a perspective view of the cannula housing assembly only with the cannula having been inserted through the patient's skin; and
Fig. 19 is a perspective view of the cannula housing assembly coupled to the tubing hub assembly with the cannula having been inserted through the patient's skin.

### DETAILED DESCRIPTION OF THE INVENTION

Certain terminology is used herein for convenience only and is not to be taken as a limitation on the present invention. The terminology includes the words specifically mentioned, derivatives thereof and words of similar import. As used herein, the term "distal" is defined to mean a direction closer to the insertion tip of a needle and cannula described herein and "proximal" is defined to mean a direction farther from the insertion tip of the needle and cannula described herein. The following describes an exemplary embodiment of the invention. However, it should be understood based on this disclosure, that the invention is not limited by the exemplary embodiment of the invention.

Referring to the figures in general, an infusion set 100 according to the present invention is disclosed. Infusion set 100 is used to insert a cannula 133 by way of an insertion needle 164 through the epidermal skin layer 52 and dermal skin layer 54 and into the fat layer 56 of a patient 50 in order to infuse medication, such as insulin, into patient 50 on an ongoing basis for a predetermined period of time, i.e., approximately 3 days. Infusion set 100 eliminates the need for multiple skin punctures to patient 50.

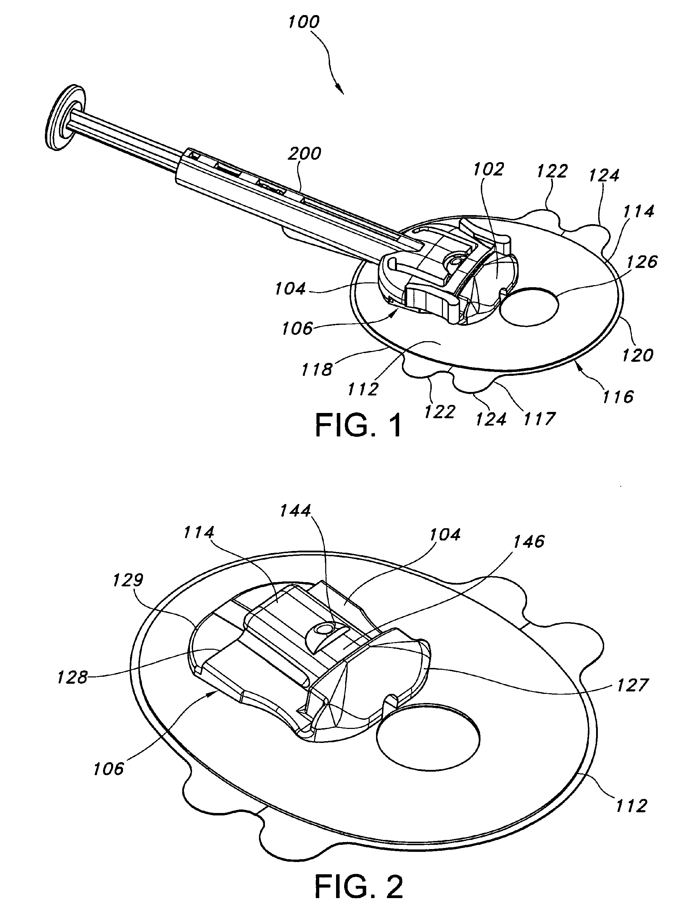

Referring now to Fig. 1, a perspective view of infusion set 100 is shown. Infusion set 100 includes a cannula housing assembly 102 and an insertion needle assembly 200 that is releasably coupled to cannula housing assembly 102. Desirably, cannula housing assembly 102 and insertion needle assembly 200 are prepackaged together as a sterilized assembly and are initially attached to a patient as a unit.

Cannula housing assembly 102 includes a cannula housing body 104 having a bottom surface 106 that is affixed to a flexible, generally planar, adhesive pad 112. As seen in Fig. 1, adhesive pad 112 is generally oblong in shape, although those skilled in the art will recognize that adhesive pad 112 may be other shapes as well.

Adhesive pad 112 includes a top surface 114 and a bottom surface 116 juxtaposed away from top surface 114. Bottom surface 116 includes a biocompatible medical grade adhesive to allow adhesive pad 112 to be affixed to the epidermal skin layer 52 of the patient 50. Adhesive backing paper 117 is releasably affixed to bottom surface 116 to prevent inadvertent adhesion of cannula housing assembly 102 with an unwanted surface. Adhesive backing paper 117 includes a proximal adhesive backing paper 118 and a distal adhesive backing paper 120 that, together, cover the entire bottom surface 116. Proximal adhesive backing paper 118 and distal adhesive backing paper 120 meet at the distal edge of cannula housing assembly 102 to allow the proximal portion of adhesive pad 112 to be affixed to the skin of patient 50 prior to insertion of cannula 133. Tear wings 122, 124 on proximal adhesive backing paper 118 and distal adhesive backing paper 120, respectively, extend beyond the outer perimeter of adhesive pad 112 to facilitate gripping and removing proximal adhesive backing paper 118 and distal adhesive backing paper 120 from adhesive pad 112.

Adhesive pad 112 also includes a transparent window 126 that may desirably offset distally from the center of adhesive pad 112. Transparent window 126 allows the person inserting infusion set 100 to observe insertion of insertion needle 164 into the patient's skin during insertion of cannula 133, and will also allow the medical personnel to observe the penetration site after insertion to check for infection or any other skin abnormality at the penetration site. Transparent window 126 may be transparent medical tape affixed over an opening in adhesive pad 112.

As can be seen from Fig. 1, cannula housing body 104 has a relatively low profile that extends above adhesive pad 112. Since cannula housing body 104 projects outwardly from the patient, the low profile is advantageous in that the amount of projection from the patient is minimized. Desirably, cannula housing body 104 is constructed from a polymer, such as polypropylene, polycarbonate, or polyurethane.

Referring to Figs. 2-4, cannula housing body 104 includes a generally curved distal surface 127 and a generally planar proximal surface 128 that each extend upward from the bottom surface 106. A top surface 125 extends longitudinally between the distal surface 127 and proximal surface 128. A bottom extension 129 extends proximally from proximal surface 128.

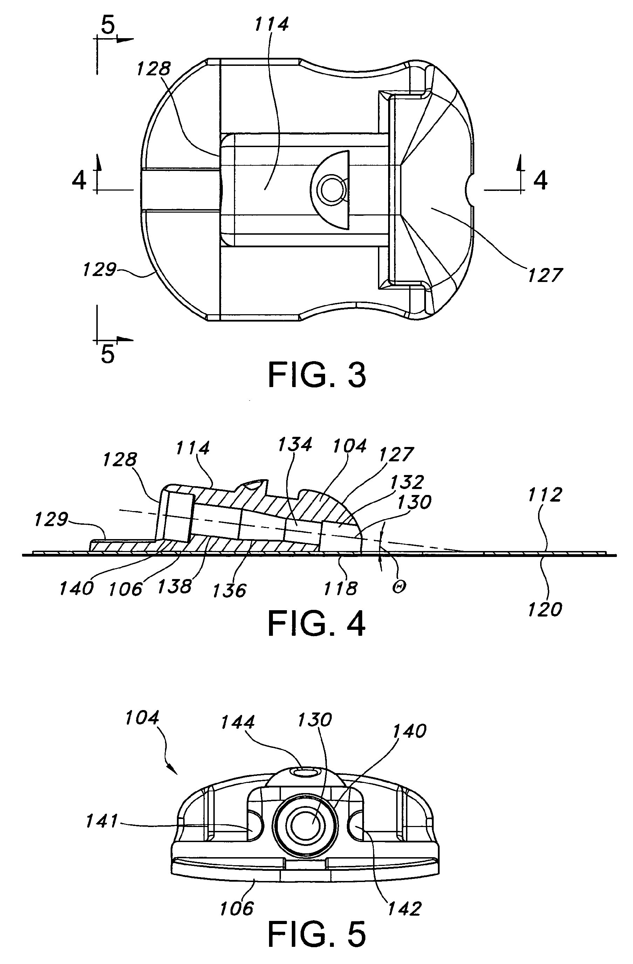

Referring now to Fig. 4, cannula housing body 104 includes a stepped bore 130 extending therethrough that narrows generally from wider to narrower in a proximal to distal direction. However, a distal-most bore portion 132 is wider than the immediately proximal bore portion 134 in order to capture and retain cannula 133 during insertion. A tapered bore portion 136 extends proximally toward a larger stepped bore portion 138, which in turn extends proximally to an even larger stepped bore portion 140 at the proximal end of cannula housing body 104.

Bore 130 extends obliquely relative to the plane of adhesive pad 112. Desirably, bore 130 extends at an angle ⊖ of between approximately 5 and 12 degrees relative to bottom surface 106 of cannula housing body 104, although those skilled in the art will recognize that bore 130 may extend at other angles. The distal end of bore 130 exits cannula housing body 104 generally at an interface between bottom surface 106 and distal surface 127, while the proximal end of bore 130 exits cannula housing body 104 generally through a central portion of proximal surface 128.

As seen in Fig. 5, parallel alignment grooves 141, 142 extend along each side of cannula housing body 104, extending in a proximal-to-distal direction. Desirably, alignment grooves 141, 142 extend at the same angle e as bore 130 with respect to bottom surface 106 in order to align insertion needle assembly 200 with cannula housing body 104 during assembly.

Referring to Figs. 2-5, a protuberance 144 extends upward from top surface 125 of cannula housing body 104. Protuberance 144 is desirably generally arcuate in shape, with a distal end of protuberance 144 being straight and a proximal end of protuberance 144 being rounded. Protuberance 144 is proximally spaced from distal surface 127 of cannula housing body 104 to form a groove 146 between protuberance 144 and distal surface 127. Desirably, as seen in Fig. 4, protuberance 144 tapers from a lower profile to a higher profile in a proximal-to-distal direction to form a raised lip at the distal end.

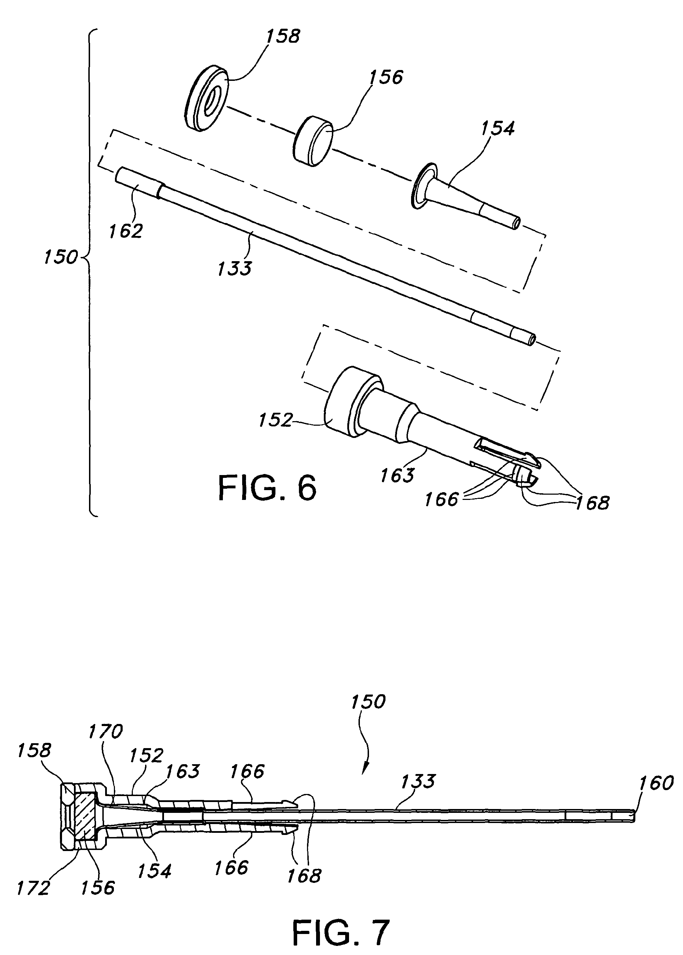

An exploded perspective view of cannula assembly 150 is shown in Fig. 6 and an assembled side sectional view of cannula assembly 150 is shown in Fig. 7. Cannula assembly 150 includes, from a distal to proximal direction, cannula 133, a cartridge body 152, a ferrule 154, an elastomeric septum 156, and a retainer 158. Cannula 133 is an elongated tubular member, having a length of desirably approximately 13-17 mm, although those skilled in the art will recognize that cannula 133 may extend greater than or less than those distances. Desirably, cannula 133 has an outer diameter of approximately 0.6 mm, although those skilled in the art will recognize that cannula 133 may have an outer diameter larger or smaller than 0.6 mm. Cannula 133 is desirably constructed from a thin-walled, biocompatible tubing, such as PTFE (Teflon®). Alternatively, cannula 133 may be constructed from a flexible metal alloy, such as nitinol, which is disclosed in copending U.S. Provisional Patent Application Serial No. 60/728,123, filed on October 19, 2005.

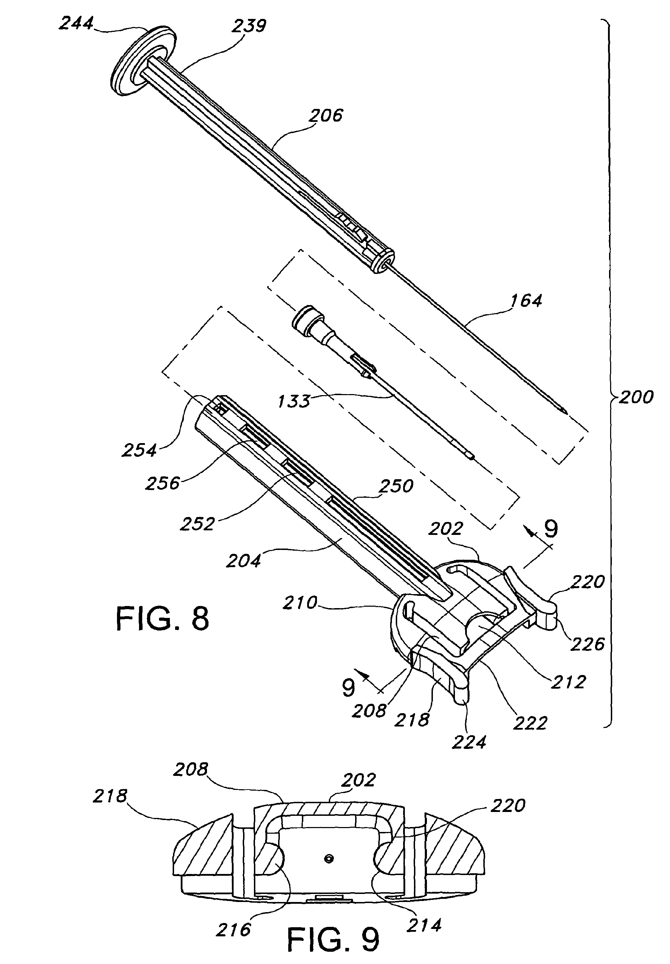

A lumen 160 extends through cannula 133. Lumen 160 is sized to allow insertion needle 164 (shown in Fig. 8) to extend therethrough and to allow insertion needle 164 to be removed from cannula 133 after cannula insertion with a minimal amount of force exerted by cannula 133 on insertion needle 164. A cannula hub 162 is located at the proximal end of cannula 133. Cannula hub 162 has a slightly larger inner diameter than cannula 133 so that cannula hub 162 may fit tightly over the distal end of ferrule 154.

Cartridge body 152 includes a generally elongated body portion 163 having a distal end with a plurality of fingers 166 extending therefrom. While three fingers 166 are shown, those skilled in the art will recognize that more or less than three fingers 166 may be used. Each finger 166 includes a barb 168 that extends therefrom. Barbs 168 are used to capture and retain cartridge body 152 within distal-most bore portion 132 of cannula housing body 104 during insertion of cannula 133. As can be seen in Fig. 7, the distal ends of fingers 166 are spaced slightly away from cannula 133 to allow fingers 166 to compress against cannula 133 and allow barbs 168 to enter the distal-most bore portion 132 of cannula housing body 104 during insertion of cannula 133.

A proximal end of body portion 163 includes a first stepped bore 170 that is sized to accommodate ferrule 154 inserted therein, and a second, larger stepped bore 172 that is sized to accommodate elastomeric septum 156 inserted therein. Once the cannula/ferrule joint is formed, cannula 133 is inserted in a proximal-to-distal direction through body portion 163 to form a fluid tight seal between ferrule 154 and cartridge body 152. Septum 156 is inserted into second bore 172, and retainer 158 is then affixed to the proximal end of cartridge body 152.

Desirably, retainer 158 is affixed to cartridge body 152 by known techniques, such as by ultrasonic welding or an adhesive. Alternatively, cartridge body 152 may be swaged or heat-staked to retain and compress septum 156 in order to eliminate the need for retainer 158. Still alternatively, instead of requiring retainer 158 to hold cannula 133 within cannula body 152, after suitable pretreatment, cannula 133 may be bonded directly to cartridge body 152.

Desirably, ferrule 154 is constructed from a polymer, such as polypropylene, or alternatively, a biocompatible metal, such as 304 stainless steel. Septum 156 is desirably constructed from a medical grade silicone rubber.

Retainer 158 is generally annularly shaped to allow insertion needle 164 to extend through retainer 158 and pierce septum 156. Desirably, retainer 158 is constructed from a polymer, such as polycarbonate or polypropylene.

Cannula assembly 150 is desirably symmetrical about a longitudinal axis of cannula assembly 150 such that cannula assembly 150 need not be inserted into cartridge body 152 in any particular alignment.

It should be noted that the above description of cannula assembly 150 illustrates only a presently preferred embodiment of cannula assembly 150. Those skilled in the art will recognize that variations of the shapes and configurations of septum 156, cannula 133, and cartridge body 152 are possible within the scope of the present invention, so long as cannula assembly 150 is sealed with septum 156, cannula 133 extends distally from cannula assembly 150, and cannula assembly 150 may be retained within cartridge body 152 by a snap-fit.

Referring now to Fig. 8, insertion needle assembly 200 includes an insertion needle body 202 and a plunger housing 204 extending proximally from insertion needle body 202. A plunger 206 is slidably disposed within plunger housing 204 and is fixedly coupled to insertion needle 164 such that translation of plunger 206 within plunger housing 204 correspondingly translates insertion needle 164 as well.

Insertion needle body 202 includes a cantilevered top face 208 that extends distally from a generally rounded proximal wall portion 210. Top face 208 includes a mating portion 212 to accommodate the protuberance 144 on top surface 125 of cannula housing body 104 when insertion needle assembly 200 is coupled to cannula housing assembly 102.

As shown in Fig. 9, first end second guide rails 214, 216 extend from under top face 208 in a proximal-to-distal direction. Guide rails 214, 216 are used to slide insertion needle assembly 200 into the corresponding grooves 141, 142 in cannula housing assembly 102. In one exemplary embodiment, guide rails 214, 216 have a generally semi-circular cross section. However, those skilled in the art will recognize that the cross sections may be any shape, so long as the corresponding grooves 141, 142 in cannula housing assembly 102 are similarly shaped to allow guide rails 214, 216 to slide along grooves 141, 142. It is desired that a clearance of approximately 0.05 mm be provided between guide rails 214, 216 and grooves 141, 142 to reduce the possibility of binding when insertion needle assembly 200 is being moved relative to cannula housing assembly 102. Guide rails 214, 216 are desirably parallel to insertion needle 164 to facilitate insertion of insertion needle 164 through septum 156 and into cannula 133.

Referring again to Figs. 8 and 9, first and second flexible arms 218, 220 extend distally from proximal wall portion 210, generally below a plane of top face 208. A resilient member 222 couples distal ends 224, 226 of first and second flexible arms 218, 220, respectively. Optionally, raised projections (not shown) may extend from distal ends 224, 226 of first and second flexible arms 218, 220 to facilitate a user's grip on first and second flexible arms 218, 220. Resilient member 222 extends generally in a plane co-planar with top face 208 and fits within groove 146 when insertion needle assembly 200 is coupled to cannula housing assembly 102. Desirably, resilient member 222 is approximately 0.75 mm thick to provide a secure locking in groove 146, yet still allow for easy release when desired. Insertion needle body 202 is desirably constructed from a flexible, resilient polymer, such as polypropylene, polycarbonate, or polyurethane.

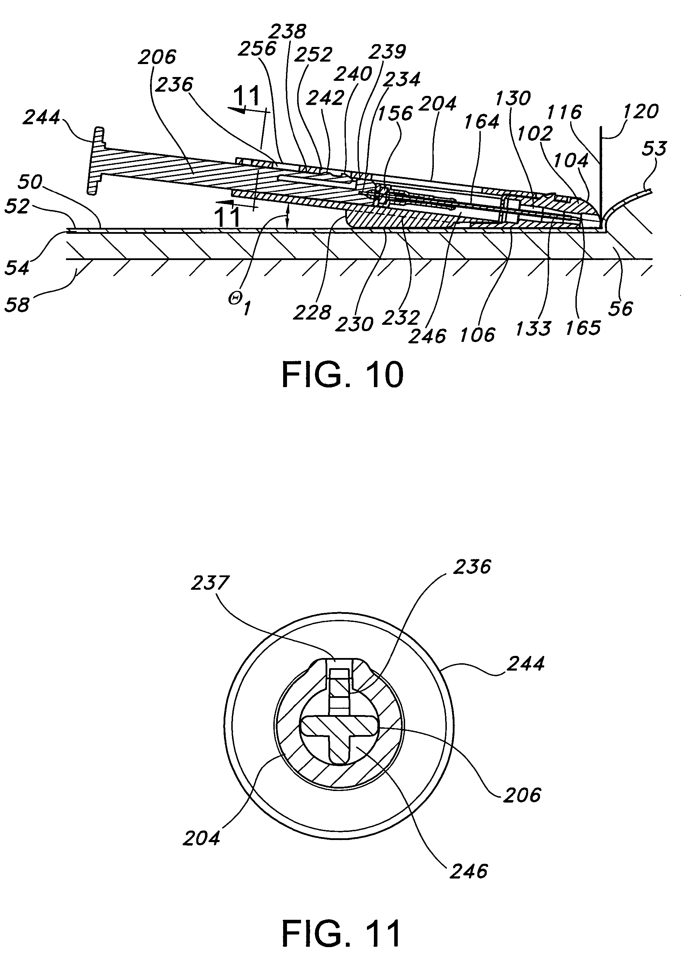

Referring to Fig. 10, plunger housing 204 extends proximally and upward from bottom surface 106 of cannula housing assembly 104 at an oblique angle ⊖₁ of between approximately 5 to 12 degrees. Desirably, ⊖₁ is the same angle as ⊖. To facilitate this angle ⊖₁, as shown in Fig. 10, an angled support member 228 extends from the bottom of plunger housing 204 at the desired oblique angle ⊖₁. Support member 228 includes a first leg 230 that is substantially co-planar with the skin surface of patient 50 and a second leg 232 that extends from first leg 230 at angle ⊖₁.

Insertion needle 164 may be a standard hollow needle beveled to a sharp point to easily penetrate septum 156 and the patient's skin. Alternatively, insertion needle 164 may be a solid trocar. A proximal end 234 of insertion needle 164 is fixedly coupled to plunger 206, such as by overmolding plunger 206 onto the proximal end of insertion needle 164. However, those skilled in the art will recognize alternative methods of coupling insertion needle 164 onto plunger 206.

Plunger 206 is an elongated member which, in one exemplary embodiment, has a generally "plus-sign" shaped cross section. Optionally, as shown in Fig. 11, one leg 236 of plunger 206 may include different dimensions than the remaining legs of plunger 206 to fit within a recess 237 in plunger housing 204. As shown in Fig. 10, a top leg 236 of plunger 206 includes a cantilevered projection 238 that extends along the distal end 239 of plunger 206. Projection 238 includes a tapered barb 240 that is disposed at the distal-most end of projection 238 and a detent 242 that is disposed proximally of barb 240.

Distal end 239 of plunger 206 includes a generally circular plunger handle 244 that has an outer diameter larger than an outer diameter of plunger housing 204. After insertion needle 164 has been inserted into patient 50, the user can grip plunger handle 244 to pull plunger 206 proximally through plunger housing 204 to remove insertion needle 164 from patient 50. Optionally, plunger 206 may be notched to allow plunger 206 to be folded into a more compact length to facilitate disposal after use. Desirably, plunger 206 is constructed from a medical grade resilient plastic, such as polypropylene or polycarbonate.

Referring again to Figs. 10 and 11, plunger housing 204 is desirably an elongated hollow member having a generally rectangular exterior cross section with a plunger bore 246 extending therethrough. Plunger bore 246 desirably includes recess 237 sized to accept one leg 236 of plunger 206 in order to prevent rotation of plunger 206 within plunger housing 204. Plunger housing 204 retains plunger 206 during insertion and also retains insertion needle 164 after insertion, when plunger 204 is in a withdrawn condition. One face of plunger housing 204, such as a top face 250, as shown in Fig. 8, includes a plurality of openings 252, 254, 256 that extend along the plunger housing 204.

A distal opening 252 engages plunger barb 240 and detent 242 to retain plunger 206 in a pre-insertion condition. In a pre-insertion condition, as shown in Fig. 10, insertion needle 164 extends through cannula 133 such that needle tip 165 extends distally of cannula 133, with both insertion needle 164 and cannula 133 extending through septum 156 and well into bore 130. It is important to note, however, that needle tip 165 does not extend distally of cannula housing assembly 102, precluding accidental needle sticks and/or contamination.

A proximal opening 254 engages plunger barb 240 to retain plunger 206 in a post-insertion condition. In the post-insertion condition, plunger 206 is retractable such that detent 242 is pulled exteriorly from plunger housing 204. A central opening 256 may be formed in plunger housing 204 to provide some frictional relief as plunger 206 is retracted. Further, central opening 256 may be used to view insertion needle 164 in the post-insertion condition to ensure that insertion needle 164 has been fully retracted from patient 50 prior to disengaging insertion needle assembly 200 from cannula housing assembly 102. However, those skilled in the art will recognize that central opening 256 is optional and may be omitted.

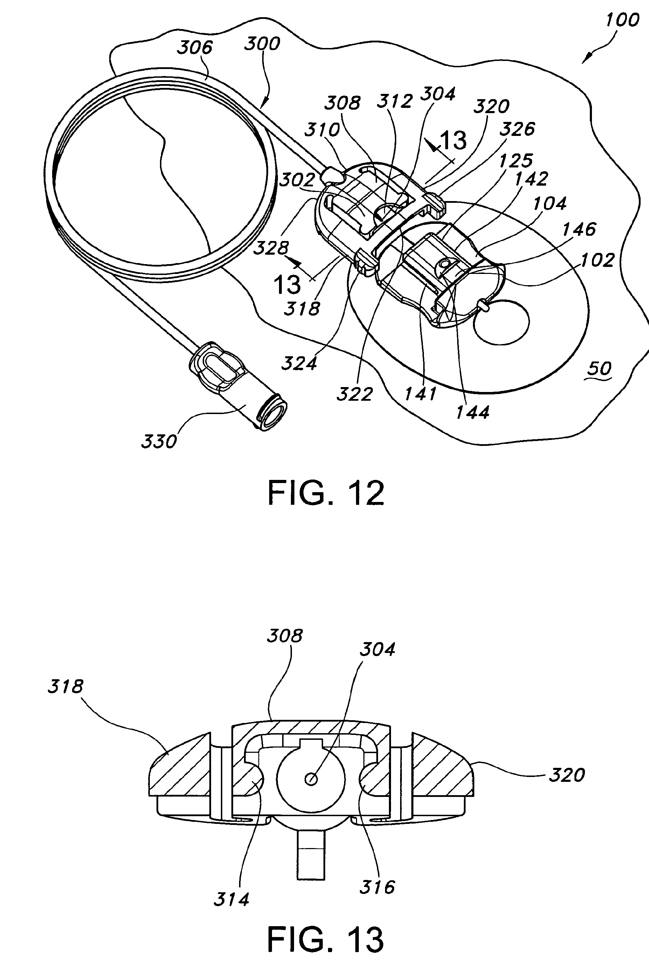

Referring now to Fig. 12, tubing hub assembly 300 is used after insertion of cannula housing assembly 104 into patient 50 to facilitate transfer of medication from a medicine dispenser, such as an insulin pump (not shown), to cannula 133 for administration to patient 50. Tubing hub assembly 300 includes a tubing hub body 302, a tubing hub needle 304, and a length of flexible tubing 306.

Tubing hub body 302 includes a cantilevered top face 308 that extends distally from a generally rounded proximal wall portion 310. Top face 308 includes a mating portion 312 to accommodate protuberance 144 on top surface 125 of cannula housing body 104 when tubing hub assembly 300 is coupled to cannula housing assembly 102. As illustrated in Fig. 13, first and second guide rails 314, 316 extend from under top face 308 in a proximal-to-distal direction. Guide rails 314, 316 are used to slide tubing hub assembly 300 into corresponding grooves 141, 142 (shown in Fig. 5) in cannula housing assembly 102. Guide rails 314, 316 desirably have the same cross sectional shape and size as guide rails 214, 216 on insertion needle body 202 in order to facilitate coupling tubing hub assembly 300 to cannula housing assembly 102.

Referring again to Fig. 12 , first and second flexible arms 318, 320 extend distally from proximal wall portion 310, generally below a plane of top face 308. A resilient member 322 couples distal ends 324, 326 of first and second flexible arms 318, 320. Resilient member 322 extends generally in a plane co-planar with top face 308. Similar to resilient member 222 on insertion needle assembly 200, resilient member 322 on tubing hub body 300 is adapted to snap into place in groove 146 on cannula housing body 104 in order to couple tubing hub body 302 to cannula housing body 104. Tubing hub body 302 is desirably constructed from a flexible, resilient polymer, such as polypropylene, polycarbonate, or polyurethane.

While the use of resilient member 322 to couple tubing hub body 302 to cannula housing body 104 is a presently preferred coupling connection, those skilled in the art will recognize that other coupling methods, such as barbs, pins, tabs, and other connections well known to those skilled in the art may be used to releasably couple tubing hub body 300 to cannula housing body 104.

Referring now only to Fig. 12, tubing hub needle 304 extends distally from tubing hub body 302 between first and second flexible arms 318, 320. Tubing hub needle 304 is sufficiently short so as to be generally encompassed on four orthogonal sides by tubing hub body 302. In this configuration, tubing hub needle 304 is well shielded to reduce the risk of accidental needle puncture or contamination when tubing hub assembly 300 is not coupled to cannula housing assembly 102. However, tubing hub needle 304 needs to be sufficiently long so as to pierce septum 156 when tubing hub assembly 300 is coupled to cannula housing assembly 102.

The length of flexible tubing 306 extends from proximal end 328 of tubing hub body 302. Tubing 306 is in fluid flow communication with tubing hub needle 304. As shown in Fig. 12, the length of tubing 306 may be sufficiently long so as to be coiled in a generally circular coil. A proximal end of tubing 306 is coupled to a connection fitting 330, such as a standard luer fitting, so that tubing 306 may be coupled to a standard infusion device, which is well known to those skilled in the art.

Desirably, the overall size of the assembled infusion set 100, with the tubing hub assembly 300 coupled to cannula housing assembly 102, is approximately 16.5 mm wide by 20 mm long by 7 mm high, although those skilled in the art will recognize that the size of infusion set 100 may be increased for easier handling without departing from the scope of the present invention.

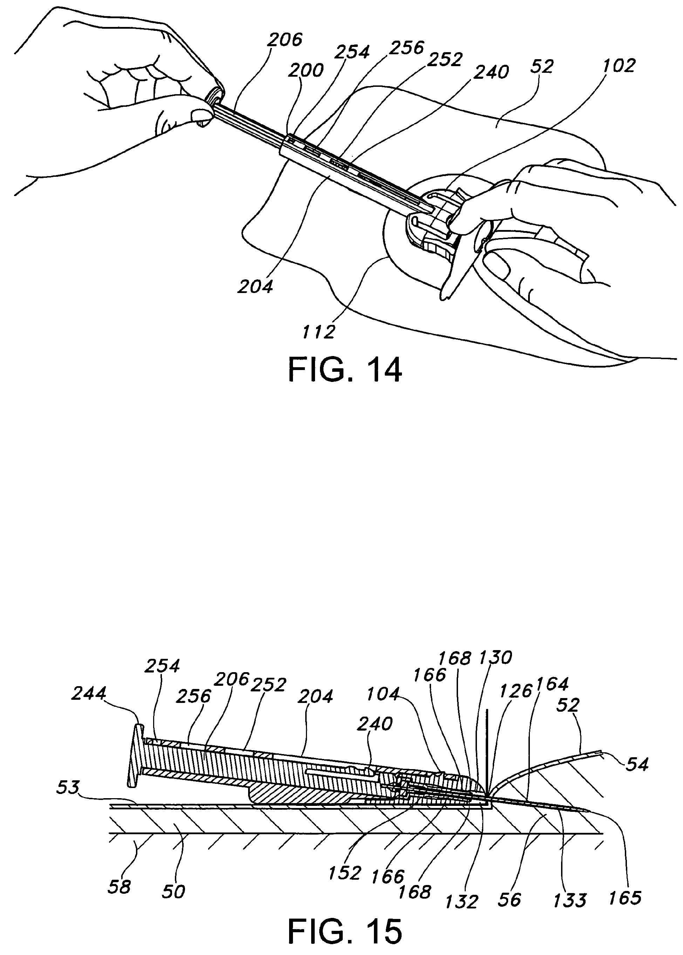

In use, cannula housing assembly 102 and insertion needle assembly 200, which are initially releasably coupled to each other, are removed from a sterilized container (not shown). Proximal adhesive backing paper 118 is removed from bottom surface 116 of adhesive pad 112 by gripping tear wings 122 and peeling proximal adhesive backing paper 118 from adhesive pad 112. Cannula housing assembly 102 is then attached to epidermal skin layer 52, as shown in Figs. 10 and 14.

A small bump 53 of tissue is formed just distally of cannula housing assembly 102 by squeezing epidermal skin layer 52. Bump 53 ensures that insertion needle 164 and cannula 133 will be inserted into subcutaneous fat tissue 56 and not underlying muscle tissue 58. It is important to note that, prior to inserting insertion needle 164 and cannula 133 through epidermal skin layer 52, both insertion needle 164 and cannula 133 are desirably housed totally within cannula housing assembly 102 and insertion needle assembly 200, thus remaining sterile.

The portion of adhesive pad 112 that is covered by distal adhesive backing paper 120 may be folded at an angle to the remainder of adhesive pad 112, as shown in Fig. 10. Such a fold allows the user to observe insertion needle 164 and cannula 133 penetrating epidermal skin layer 52 during insertion.

Cannula housing assembly 102 and insertion needle assembly 200 are desirably gripped by the user as shown in Fig. 14. Plunger 206 is advanced distally through plunger housing 204 to the location shown in Fig. 15. As plunger 204 is advanced, plunger barb 240 is forced from distal opening 252 into plunger housing 204. Advancement of plunger 206 also advances needle 164 and cannula 133 together. Needle tip 165 punctures window 126 and pierces epidermal skin layer 52 and dermal skin layer 54, and stops in fat layer 56. As insertion needle 164 and cannula 133 are advanced, fingers 166 of cannula cartridge body 152 are compressed toward cannula 133 as fingers 166 advance through bore 130 of cannula housing body 104. As fingers 166 enter distal-most bore portion 132, fingers 166 expand away from cannula 133, and barbs 168 on fingers 166 prevent cannula 133 from being retracted proximally into bore 130. At this point, plunger handle 244 has engaged proximal end of plunger housing 204 and cannula housing body 104 has been advanced into bore 130 as far distally as cannula housing body 104 can be advanced, thus precluding further distal advancement of cannula 133 into patient 50.

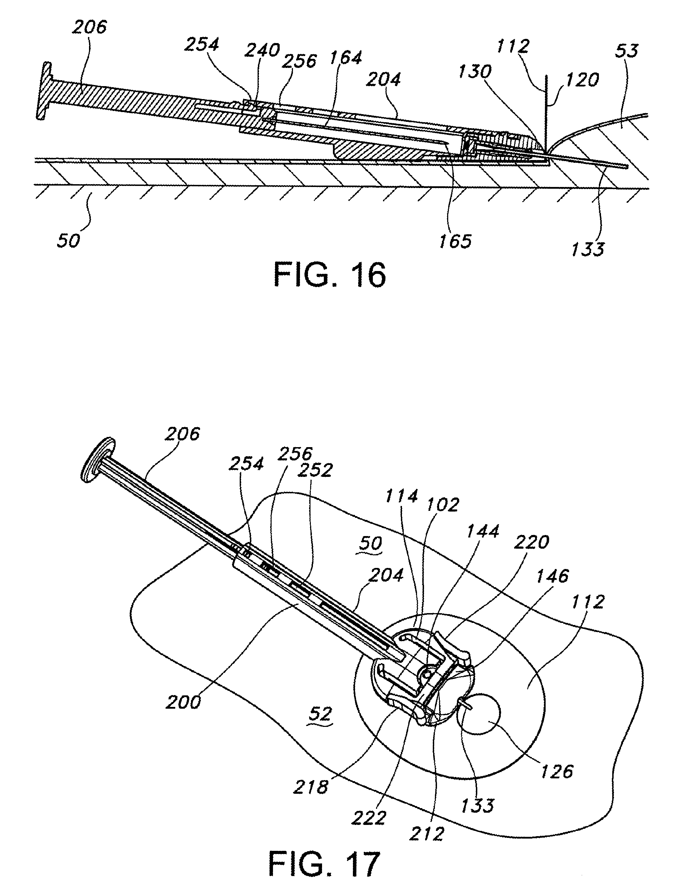

Plunger 206 is next withdrawn proximally through plunger housing 204 to the position shown in Fig. 16, withdrawing insertion needle 164 from patient 50 and through bore 130 of cannula housing body 104, leaving cannula 133 inserted in patient 50. Plunger 206 is withdrawn until plunger barb 240 engages proximal opening 254. The user views insertion needle 164 through central opening 256 to ensure that insertion needle 156 is fully withdrawn from the patient. At this point, needle tip 165 is safely housed within plunger body 204, eliminating the potential for an accidental needle stick.

The user next releases bump 53 of tissue and removes distal adhesive backing paper 120 from adhesive pad 112. The portion of adhesive pad 112 that was covered by distal adhesive backing paper 120 may now be smoothed over the insertion site, as shown in Fig. 17. Preferred insertion sites include the abdomen and the upper thigh. Since needle 164 and cannula 133 punctured window 126, the insertion location of the cannula into epidermal skin layer 52 may be observed through window 126 for any abnormalities, such as post-insertion swelling or infection surrounding the insertion site. After the user releases bump 53 of tissue, it may be noticed that cannula 133 bends slightly before penetrating epidermis 52. The flexibility of cannula 133 allows cannula 133 to bend in this manner without kinking or collapsing.

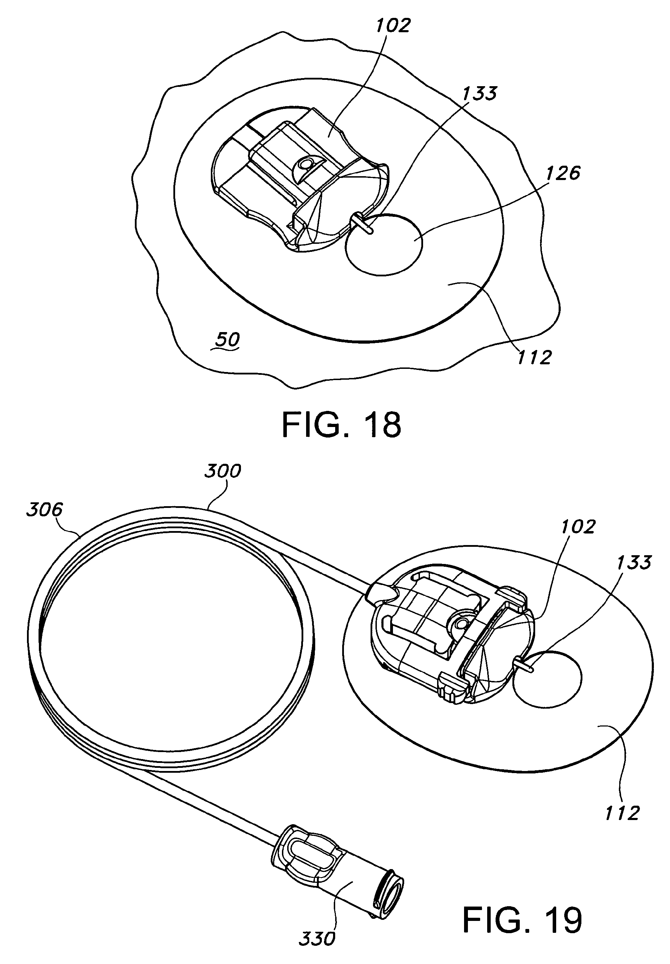

Flexible arms 218, 220 of insertion needle assembly 200 are next compressed toward cannula housing assembly 102, lifting resilient member 222 out of groove 146 and over protuberance 144 on top surface 125 of cannula housing assembly 102. Insertion needle assembly 200 may now be removed from cannula housing assembly 102 by moving insertion needle assembly 200 proximally relative to cannula housing assembly 102. Insertion needle assembly 200 may now be discarded. As shown in Fig. 18, cannula housing assembly 102 is now ready to accept tubing hub assembly 300.

Referring back to Fig. 12, to use infusion set 100, tubing hub assembly 300 must be coupled to cannula housing assembly 102. To couple tubing hub assembly 300 to cannula housing assembly 102, tubing hub guide rails 314, 316 are aligned with grooves 141, 142 on the sides of cannula housing assembly 102.
Tubing hub assembly 300 is advanced distally along grooves 141, 142 until resilient member 322 slides up and over protuberance 144 and snaps into place in groove 146, releasably coupling tubing hub assembly 300 to cannula housing assembly 102. Desirably, the snapping of resilient member 322 into groove 146 results in a click or snapping sound to audibly indicate that a secure connection has been made between tubing hub assembly 300 and cannula housing assembly 102.

As tubing hub assembly 300 is being advanced proximally, tubing hub needle 304 advances toward and then pierces septum 156, providing fluid flow communication between cannula 133 and tubing 306. A perspective view of tubing hub assembly 300 coupled to cannula housing assembly 102 is shown in Fig. 19. Medication may now be infused through tubing 306 and tubing hub needle 304, through cannula 133, and into patient 50.

In the event that tubing hub assembly 300 must be removed from cannula housing assembly 102, such as for patient 50 to bathe or to replace a damaged tubing 306, flexible arms 318, 320 of tubing hub assembly 300 are compressed toward cannula housing assembly 102, lifting resilient band 322 out of groove 146 and over protuberance 144 on top surface 125 of cannula housing assembly 102. Tubing hub assembly 300 may now be removed from cannula housing assembly 102 by moving tubing hub assembly 300 proximally relative to cannula housing assembly 102. Tubing hub assembly 300 may now be discarded and a replacement tubing hub assembly may be installed as described above.

While the present invention is intended to be used for the infusion of medication, such as insulin, those skilled in the art will recognize that the present invention may also be used for fluid withdrawal as well.

Although the invention is illustrated and described herein with reference to a specific embodiment, the invention is not intended to be limited to the details shown. Rather, various modifications may be made in the details within the scope and range of equivalents of the claims and without departing from the invention.

## Claims

1. An infusion set comprising:
a cannula hub assembly including a cannula housing body having a distal end and a bottom surface; and
an insertion needle assembly including:
an insertion needle hub adapted to be coupled to the cannula housing body,
a cannula having a distal cannula end, wherein the cannula is at least partially slidably disposed within the insertion needle hub, and
an insertion needle having a distal insertion needle end, wherein the insertion needle extends coaxially through the cannula,
wherein the distal cannula end and the distal insertion needle end are adapted to be inserted together through the distal end of the cannula housing body, and the insertion needle is retractable from the cannula housing body to be at least substantially disposed within the insertion needle hub.

2. The infusion set according to claim 1, wherein the cannula housing body includes a bore extending longitudinally therethrough and wherein the insertion needle is slidably disposable through the bore.

3. The infusion set according to claim 2, wherein the base extends in a plane and wherein the bore extends obliquely relative to the plane.

4. The infusion set according to claim 3, wherein the bore extends at an angle between approximately 5 and 12 degrees relative to the plane.

5. The infusion set according to claim 1, further comprising a tubing hub assembly adapted to releasably couple to the cannula hub assembly.

6. The infusion set according to claim 1, further comprising a cartridge body coupled to a proximal end of the cannula and wherein the cartridge body comprises at least one barb extending therefrom, wherein the at least one barb is adapted to retain the cartridge body in the cannula housing body.

7. The infusion set according to claim 1, further comprising means for advancing the insertion needle and the cannula distally through the cannula housing body and for proximally retracting the insertion needle such that the insertion needle may be substantially disposed within the insertion needle assembly.

8. The infusion set according to claim 7, wherein the means for advancing the insertion needle and the cannula comprises a plunger coupled to the insertion needle and slidably disposed at least partially within the insertion needle assembly.

9. The infusion set according to claim 8, wherein the insertion needle hub further comprises a first detent disposed to retain the plunger in a retracted position and a second detent, located proximally of the first detent, disposed to retain the plunger in a withdrawn position.

10. The infusion set according to claim 1, wherein the insertion needle is retractable from the cannula housing body to be entirely disposed within the insertion needle hub.

11. A method of percutaneously inserting a cannula of an infusion set through a patient's skin, wherein the infusion set comprises an insertion needle hub releasably coupled to a cannula hub assembly and wherein the infusion set further comprises the cannula having an insertion needle disposed therethrough, the needle extending slidably within the insertion needle assembly and the cannula hub assembly, the method comprising:
attaching at least a portion of a base portion of an infusion set to the patient's skin at an insertion site;
advancing the cannula and the insertion needle through the cannula hub assembly and through the patient's skin; and
retracting the insertion needle into the insertion needle assembly while leaving the cannula in the patient's skin.

12. The method according to claim 11, further comprising, after retracting the insertion needle, releasing the insertion needle hub from the cannula hub assembly.

13. The method according to claim 12, further comprising, after releasing the insertion needle hub from the cannula hub assembly, connecting a tubing hub assembly to the cannula hub assembly.

14. The method according to claim 11, further comprising, prior to displacing the cannula and the insertion needle through the cannula hub assembly and through the patient's skin, forming a fold of skin tissue at the insertion site.

15. The method according to claim 11, wherein displacing the cannula and the insertion needle through the cannula hub assembly and through the patient's skin comprises piercing the base portion.

16. The method according to claim 15, further comprising, after piercing the base portion, attaching a remaining portion of the base portion to the patient's skin.

17. The method according to claim 11, wherein retracting the insertion needle into the insertion needle assembly comprises locking the insertion needle with respect to the insertion needle assembly.

18. A safety needle assembly for an infusion set comprising:
a cannula housing body having a bore extending therethrough;
an insertion needle hub body releasably connected to the cannula housing body;
a cannula having a distal cannula end, wherein the cannula is at least partially slidably disposed within the insertion needle hub body;
an insertion needle having a distal insertion needle end, wherein the needle extends coaxially through the cannula; and
a plunger in operative contact with the insertion needle, wherein the plunger advances the insertion needle and the cannula distally through the cannula housing body and proximally retracts the insertion needle such that the insertion needle is entirely disposed within the insertion needle hub body.

19. The safety needle assembly for an infusion set according to claim 18, wherein the plunger is fixedly connected to the insertion needle and slidably disposed at least partially within the insertion needle hub body.

20. The safety needle assembly for an infusion set according to claim 19, wherein the insertion needle hub body further comprises a first detent disposed to retain the plunger in a retracted position and a second detent, located proximally of the first detent, disposed to retain the plunger in a withdrawn position.
